# EUROPEAN PATENT APPLICATION

(11) **EP 4 692 321 A1**
(43) Date of publication of application: **11.02.2026**
(21) Application number: 24796949.6
(22) Date of filing: 19.04.2024
(51) Int. Cl.: C12N 5/071, C12N 5/00, C12N 5/077

(54) **CELL STRUCTURE PRODUCTION METHOD**

(30) Priority: 27.04.2023 JP 2023073301
(71) Applicant: Toppan Holdings Inc., Tokyo 110-0016 (JP); The University of Osaka, Osaka 565-0871 (JP)
(72) Inventor: HATTORI, Koichi, Tokyo 110-0016 (JP); HIRAOKA, Yasuyuki, Tokyo 110-0016 (JP); MATSUSAKI, Michiya, Suita-shi, Osaka 565-0871 (JP)
(74) Representative: Hasegawa, Kan
(86) International application number: PCT/JP2024/015644
(87) International publication number: WO 2024/225202

(57) **Abstract**

The present invention relates to a method for producing a cell structure including a step of culturing a plurality of cells, including at least epithelial cells and vascular endothelial cells, in a medium including one or more growth factors involved in angiogenesis.

## Description

### Technical Field

The present invention relates to a method for producing a cell structure.

### Background Art

Various studies have been conducted so far on methods for producing cell structures in which blood vessels are formed (see, for example, Patent Literature 1). Patent Literature 1 discloses a method for producing a three-dimensional cell culture having a microvascular-like structure, the method including predetermined Processes 1 to 3. Process 1 in the production method is a process of seeding agitated fibroblasts into a culture vessel to form a first fibroblast layer, Process 2 is a process of adding collagen IV onto the first fibroblast layer, then seeding and culturing vascular endothelial cells to form a vasculature-like structure layer on the first fibroblast layer, and Process 3 is a process of seeding agitated fibroblasts together with VEGF and FGF onto the vasculature-like structure layer to form a second fibroblast layer in which a vasculature-like structure extends upward from the vasculature-like structure layer.

Further, for example, Patent Literature 2 discloses a biologically active placental-derived liquid substrate (hpS) containing extracellular matrix (ECM) proteins, cytokines, and growth factors. It has also been described that in an experiment in which HUVECs were seeded on hpS, HUVECs were detected as assembled into an interconnected cellular network (vasculogenesis).

### Citation List

### Patent Literature

[Patent Literature 1] Japanese Unexamined Patent Publication No. 2017-176025
[Patent Literature 2] Japanese Unexamined Patent Publication No. 2022-527648

### Summary of Invention

### Technical Problem

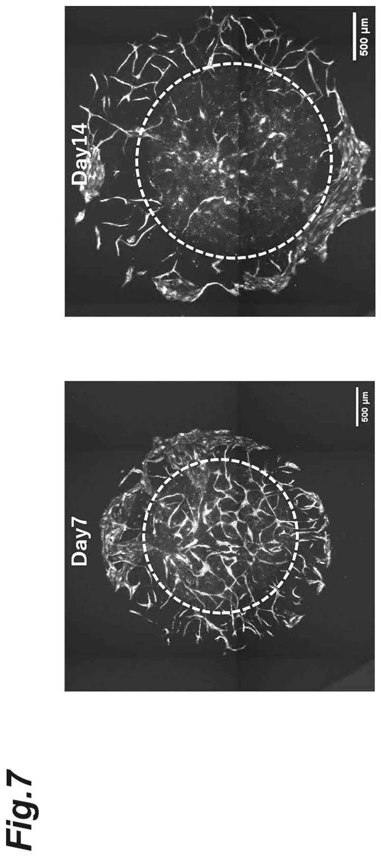

The present inventors have newly discovered that, in conventional liver models, the presence of certain non-parenchymal cells (hepatic stellate cell), which were previously considered to enhance liver function through co-culture, actually leads to a failure to maintain liver function in the liver model, whereas the absence of hepatic stellate cells results in a failure to form blood vessels (see Test Examples 3 and 1). In addition, even if blood vessels are formed in a liver model lacking hepatic stellate cells, there are cases where the blood vessels may diffuse outside the cell structure during culturing (see reference example and FIG. 7). As described above, sufficient studies have not been conducted on the method for producing a cell structure which includes epithelial cells and in which blood vessels that do not diffuse to the outside are formed.

An object of the present invention is to provide a method for producing a cell structure which includes epithelial cells and in which blood vessels that do not diffuse to the outside are formed.

### Solution to Problem

The present inventors have found that cell structures in which blood vessels that do not diffuse to the outside are formed can be produced by culturing hepatocytes and vascular endothelial cells in a medium including VEGF-A. Based on this finding, it is considered that the technique is also applicable to the production of a cell structure which includes epithelial cells and in which blood vessels that do not diffuse to the outside are formed. The present invention is based on this novel finding.

That is, the present invention encompasses the following inventions.
[1] A method for producing a cell structure, comprising a step of culturing a plurality of cells, comprising at least epithelial cells and vascular endothelial cells, in a medium comprising one or more growth factors involved in angiogenesis.
[2] The method according to [1], in which the medium comprises two or more growth factors involved in angiogenesis, and
   the two or more growth factors involved in angiogenesis comprise a vascular development factor and a vascular maintenance factor.
[3] The method according to [1] or [2], in which a content of the growth factor involved in angiogenesis in the medium is 10 ng/mL or more.
[4] The method according to any one of [1] to [3], in which the step of culturing is performed under conditions that allow a medium component to be taken up from all directions of the cell structure.
[5] The method according to any one of [1] to [4], in which the epithelial cells are hepatocytes and the cells do not comprise hepatic stellate cells.
[6] The method according to any one of [1] to [5], further including prior to the step of culturing, a step of obtaining a mixture comprising the plurality of cells and an extracellular matrix component.
[7] The method according to [6], in which the extracellular matrix components comprise collagen components.
[8] The method according to [6] or [7], in which the mixture further comprises a polymer electrolyte.
[9] The method according to [8], in which the polymer electrolyte comprises heparin.

### Advantageous Effects of Invention

The present invention provides a method for producing a cell structure which includes epithelial cells and in which blood vessels that do not diffuse to the outside are formed.

### Brief Description of Drawing

FIG. 1 is an image illustrating a result of evaluation of angiogenesis in a cell structure.
FIG. 2 is an image illustrating the result of evaluation of the angiogenesis in the cell structure.
FIG. 3 is an image illustrating the result of evaluation of the angiogenesis in the cell structure.
FIG. 4 is a graph illustrating a result of analysis of blood vessel length in the cell structure.
FIG. 5 is a graph illustrating a result of analysis of CYP3A4 gene and CYP2C9 gene expression levels in the cell structure.
FIG. 6 is an image illustrating a result of evaluation of angiogenesis in the cell structure.
FIG. 7 is an image illustrating the result of evaluation of the angiogenesis in the cell structure.
FIG. 8 is an image illustrating a result of observation of vascular structures in the cell structure.
FIG. 9 is an image illustrating the result of observation of the vascular structures in the cell structure.
FIG. 10 is a graph illustrating a result of analysis of blood vessel length in the cell structure.
FIG. 11 is a graph illustrating a result of analysis of cell viability in the cell structure.
FIG. 12 is a graph illustrating a result of analysis of CYP3A4 activity in the cell structure.

### Description of Embodiment

Hereinafter, embodiments of the present invention will be described in detail. However, the present invention is not limited to the following embodiments.

### [Method for Producing Cell Structure]

In the present specification, the "cell structure" means a cell aggregate (mass-like cell population) in which a plurality of cells are three-dimensionally disposed, and is an aggregate artificially produced by cell culture. The cells contained in the cell structure may be of one type or two or more types.

The shape of the cell structure is not particularly limited, and examples thereof include a spherical shape, substantially a spherical shape, an ellipsoidal shape, substantially an ellipsoidal shape, a hemispherical shape, substantially a hemispherical shape, a semicircular shape, substantially a semicircular shape, a rectangular parallelepiped shape, and substantially a rectangular parallelepiped shape. Here, the living tissue includes sweat glands, lymphatic vessels, sebaceous glands, and the like, and has a more complex configuration than the cell structure. Therefore, the cell structure and the living tissue can be easily distinguished from each other. In addition, the cell structure may be aggregated in a mass in which the cell structure is adhered to a support or aggregated in a mass in which the cell structure is not adhered to a support.

The method for producing a cell structure according to the present embodiment includes a step of culturing a plurality of cells including at least epithelial cells and vascular endothelial cells in a medium including one or more growth factors involved in angiogenesis (culturing step). In the present specification, "culturing cells" means maintaining the cells in the medium under conditions that do not cause cell death or deterioration. The culturing of cells may or may not involve cell proliferation.

The method for producing a cell structure according to the present embodiment, by having the above configuration, a cell structure which includes epithelial cells and in which blood vessels that do not diffuse to the outside are formed can be manufactured. The term "cell structure in which blood vessels are formed" means a cell structure having a tubular structure formed by vascular endothelial cells. The blood vessels can be confirmed, for example, by immunohistochemical staining or microscopic observation using vascular endothelial cells that express fluorescent proteins. The formation of blood vessels may, for example, be the formation of blood vessels to such an extent that, when the cell structure is observed from above under a microscope, it is observed to have a plurality of branch points and a mesh-like structure.

The term "cell structure in which blood vessels that do not diffuse to the outside are formed" may refer to a cell structure having blood vessels that are not separated from the cell structure even after on Day 14 of culturing. In addition, as will be shown in the reference example described later, where blood vessels separated from the cell structure were observed outside the cell structure, the term may also refer to a case in which, upon microscopic observation of the cell structure from above on Day 14 of culturing, no blood vessels separated from the cell structure are observed outside the cell structure.

In the culturing step, a plurality of cells including at least epithelial cells and vascular endothelial cells are cultured in a medium including one or more growth factors involved in angiogenesis, thereby obtaining a cell structure which includes epithelial cells and in which blood vessels that do not diffuse to the outside are formed.

Epithelial cells are cells that form epithelial tissues present in the skin, digestive organs, blood vessels, and the like. Examples of the epithelial cells used in the culturing step include, for example, hepatocytes, epidermal keratinocytes, renal epithelial cells, vascular epithelial cells, alveolar epithelial cells, intestinal epithelial cells, retinal epithelial cells, neuroepithelial cells, gingival epithelial cells, bile duct epithelial cells, and thymic epithelial cells, with hepatocytes being preferred.

Hepatocytes are also referred to as hepatic parenchymal cells, and are, for example, cells having functions such as bile secretion and plasma protein secretion. The hepatocytes constituting the cell structure may be primary hepatocytes collected from the liver of an animal, cells obtained by culturing primary hepatocytes, cultured cell lines in which primary hepatocytes are established, or hepatoblasts artificially differentiated from stem cells. Examples of primary hepatocytes include primary human hepatocytes such as PXB Cells. Examples of cultured cell lines include cell lines derived from inactivated liver cancer cells such as HepG2. Examples of stem cells that differentiate into hepatoblasts include embryonic stem cells (ES cells), induced pluripotent stem cells (iPS cells), and mesenchymal stem cells. The hepatocytes contained in the cell structure are preferably non-cancerous cells such as primary hepatocytes and hepatoblasts, and more preferably PXB Cell in consideration of ease of handling.

The epithelial cells in the culturing step may be of one type or two or more types. For example, in a case where the epithelial cells in the culturing step are hepatocytes, the hepatocytes may include a plurality of hepatocytes having different genotypes of proteins involved in liver function. Conversely, all of the hepatocytes to be cultured may have the same genotype of proteins involved in liver function. Examples of proteins involved in liver functions include drug-metabolizing enzymes.

In the culturing step, the ratio (X1/X0 × 100) of the number (X1) of epithelial cells to the total number (X0) of the plurality of cells to be cultured may be 5% or more, 10% or more, 15% or more, 20% or more, 25% or more, 30% or more, 35% or more, 40% or more, 45% or more, 50% or more, 55% or more, 60% or more, or 65% or more, and may be 95% or less, 90% or less, 80% or less, or 75% or less. In the culturing step, the ratio (X1/X0 × 100) of the number (X1) of epithelial cells to the total number (X0) of the plurality of cells to be cultured may preferably be, from the viewpoint of being more suitable as a cell structure including epithelial cells, from 60% to 80% or from 60% to 70%, and may also be from 60% to 95%, from 65% to 95%, from 65% to 90%, from 75% to 90%, or from 80% to 88%. In a case where the epithelial cells are hepatocytes, it is desirable that the ratio of hepatocytes be 50% or more, which is a condition close to that in vivo.

Vascular endothelial cells means squamous cells that constitute the surface of the intravascular lumen. Vascular endothelial cells in the culturing step may be, for example, sinusoidal endothelial cells or human umbilical vein endothelial cells (HUVECs). The sinusoidal endothelial cells are hepatic non-parenchymal cells (cells other than hepatocytes among cells constituting the liver), and are cells which have a large number of small pore aggregates (cribriform plate structure) in the cytoplasm, and have a characteristic morphology different from other vascular endothelial cells such as lacking of a basement membrane. The vascular endothelial cells used in the culturing step may be primary cells (primary vascular endothelial cells) collected from the liver of an animal (for example, a human), cells obtained by culturing primary cells, cultured cell lines established from primary cells, or cells artificially differentiated from stem cells. The cultured cell lines may be established by gene introduction, and specific examples include cultured cell lines into which immortalizing genes such as hTERT or SV40LT have been introduced. Examples of primary vascular endothelial cells include primary sinusoidal endothelial cells such as Product model No. 5000 (commercially available from Sciencell). In a case where a cultured cell line immortalized by introducing a gene into primary sinusoidal endothelial cells is used, the effects of angiogenesis and maintenance by growth factors involved in angiogenesis are expected to be further enhanced. Examples of cultured cell lines include cultured cell lines such as Product model No. T0056 (commercially available from Applied Biological Materials). Examples of differentiated stem cells include embryonic stem cells (ES cells) and induced pluripotent stem cells (iPS cells). The vascular endothelial cells used in the culturing step may be non-cancerous cells.

In the culturing step, the ratio (X2/X0 × 100) of the number (X2) of vascular endothelial cells to the total number (X0) of the plurality of cells to be cultured may be 5% or more, 10% or more, 12% or more, 14% or more, 15% or more, 20% or more, or 25% or more, and may be 50% or less, 45% or less, 40% or less, 35% or less, 30% or less, 25% or less, 20% or less, or 18% or less. In the culturing step, the ratio (X2/X0 × 100) of the number (X2) of vascular endothelial cells to the total number (X0) of the plurality of cells to be cultured may preferably be, from the viewpoint of being more suitable as a cell structure having blood vessels, from 5% to 40%, from 5% to 35%, from 10% to 35%, from 10% to 25%, or from 12% to 20%, and may also be from 20% to 40% or from 30% to 40%.

The plurality of cells used in the culturing step may include cells other than epithelial cells and vascular endothelial cells, as long as the effects of the present invention are not impaired. The other cells may be, for example, matured somatic cells, or undifferentiated cells such as stem cells. Specific examples of somatic cells include, for example, nerve cells, dendritic cells, immune cells, lymphatic endothelial cells, fibroblasts, cardiomyocytes, pancreatic islet cells, smooth muscle cells, bone cells, and spleen cells. Examples of stem cells include ES cells, iPS cells, and mesenchymal stem cells. The other cells may be normal cells or cells in which any cell function is enhanced or inhibited such as cancer cells. "Cancer cells" are cells derived from somatic cells and having an acquired infinite proliferative ability.

The hepatic stellate cells are hepatic non-parenchymal cells (cells other than hepatocytes among cells constituting the liver), have a function of storing vitamin A, and are present in the space of Disse, which is an area between hepatocytes and the sinusoid in the liver. In conventional culture systems, hepatic stellate cells were believed to further enhance liver function when co-cultured with hepatocytes. However, it has been found that hepatic stellate cells isolated from the body or cell lines established from such hepatic stellate cells become activated under in vitro culture conditions, and in a case where co-cultured with hepatocytes, the hepatic stellate cells exert adverse effects, such as a significant decrease in the expression levels of hepatocyte-specific metabolic enzymes as culturing progresses, and that, in the absence of hepatic stellate cells, blood vessels are not formed. Therefore, in a case where the epithelial cells in the culturing step are hepatocytes, it is preferable not to contain hepatic stellate cells that exhibit an activated state as cells other than epithelial cells and vascular endothelial cells. Cells that exhibit such an activated state include, for example, hepatic stellate cell lines such as LX2.

The origin of epithelial cells, vascular endothelial cells, or the other cells contained in the cell structure is not particularly limited, and may be, for example, cells derived from mammals such as humans, monkeys, dogs, cats, rabbits, pigs, cows, mice, or rats.

The total number (X0) of cells cultured in the culturing steping is not particularly limited, and may be appropriately determined in consideration of factors such as the thickness and shape of the cell structure to be constructed and the size of the cell culture vessel used for construction. The total number (X0) of a plurality of cells cultured in the culturing step may be 1 × 10³ cells or more, 1 × 10⁴ cells or more, 1 × 10⁵ cells or more, or 1 × 10⁶ cells or more. The total number (X0) of a plurality of cells cultured in the culturing step may be 1 × 10⁹ cells or less, 1 × 10⁸ cells or less, or 1 × 10⁷ cells or less. Further, for example, the cell density in the medium during the culturing steping may be 10³ to 10⁷ cells/mL, or 10⁴ to 10⁶ cells/mL.

The culture conditions for the plurality of cells in the culturing step may be any conditions under which the cells do not die or deteriorate, and may be appropriately set according to the type of cells. For example, the culture temperature may be 20°C to 40°C, or 30°C to 37°C. The pH of a medium may be 6 to 8 or may be 7.2 to 7.4. The culture time may be 1 day to 2 weeks, or 1 week to 2 weeks.

The medium in the culturing step contains one or more growth factors involved in angiogenesis. The "growth factors involved in angiogenesis" means growth factors that act at any stage in the step of forming, maturing, or maintaining new blood vessels either from pre-existing blood vessels or in regions where no blood vessels previously existed. The "growth factor" is a general term for endogenous proteins that promote the proliferation or differentiation of specific cells. The growth factors involved in angiogenesis may contain at least a vascular development factor or a vascular maintenance factor. The "vascular development factor" means a growth factor among the growth factors involved in angiogenesis, which acts at an early stage of angiogenesis, that is, a growth factor having an action of promoting vascular network formation by vascular endothelial cells. The "vascular maintenance factors" will be described below.

The growth factors involved in angiogenesis contained in the medium may be two or more, three or more, four or more, five or more, six or more, six or more, seven or more, eight or more, nine or more, ten or more, eleven or more, twelve or more, or thirteen or more.

The medium used in the culturing step preferably contains two or more growth factors involved in angiogenesis, and in such a case, it is more preferable that the growth factors involved in angiogenesis include a vascular development factor and a vascular maintenance factor. The "vascular maintenance factor" means a growth factor among the growth factors involved in angiogenesis that acts in a later stage of angiogenesis, that is, during the stage of maintaining the formed blood vessels, and has an effect of stabilizing and maintaining the vascular structure. In a case where the medium in the culturing step contains a vascular development factor and a vascular maintenance factor as growth factors involved in angiogenesis, not only are blood vessels that do not diffuse to the outside formed in the cell structure, but also the degeneration of the formed blood vessels is suppressed.

Examples of vascular development factors include, for example, vascular endothelial growth factor (VEGF), fibroblast growth factor (FGF), and the like. Examples of VEGF include, for example, VEGF-A to VEGF-E. Examples of FGF include, for example, FGF1 to FGF10.

Examples of vascular maintenance factors include, for example, angiopoietins (for example, angiopoietin-1 to angiopoietin-4), insulin, hemopexin, epidermal growth factor (EGF), melanotransferrin, heparan sulfate proteoglycan (HSPG2), insulin-like growth factor-1 (IGF-1), and leucine-rich alpha-2-glycoprotein 1 (LRG1), and the like. The vascular maintenance factor may be at least one selected from the group consisting of the above-described vascular maintenance factors.

From the viewpoint of further promoting angiogenesis, the concentration of the growth factor involved in angiogenesis in the medium may be, for example, 10 ng/mL or more, 20 ng/mL or more, 30 ng/mL or more, 40 ng/mL or more, 50 ng/mL or more, 60 ng/mL or more, 70 ng/mL or more, 80 ng/mL or more, 90 ng/mL or more, or 100 ng/mL or more. Also, the concentration of the growth factor involved in angiogenesis in the medium may be, for example, 1,000 ng/mL or less, 900 ng/mL or less, 800 ng/mL or less, 700 ng/mL or less, 600 ng/mL or less, 500 ng/mL or less, 400 ng/mL or less, 300 ng/mL or less, or 200 ng/mL or less.

In a case where both vascular development factors and vascular maintenance factors are contained in the medium, the ratio of the concentration (ng/mL) of the vascular maintenance factor in the medium to the concentration (ng/mL) of the vascular development factor in the medium may be, for example, 0.5 or more, 0.6 or more, 0.7 or more, 0.8 or more, 0.9 or more, 1 or more, 2 or more, 3 or more, or 4 or more, and may also be 20 or less, 15 or less, 10 or less, 9 or less, 8 or less, 7 or less, or 5 or less.

The medium is not particularly limited as long as it contains the above growth factors involved in angiogenesis, and an appropriate medium can be selected according to the type of cells to be cultured. Examples of the medium include Eagle's MEM medium, DMEM, Modified Eagle medium (MEM), Minimum Essential medium, RPMI, GlutaMax medium, and hepatocyte-specific media (for example, HCM medium manufactured by Lonza). The medium may be a medium to which serum is added, or may be a serum-free medium. Furthermore, the medium may be a mixed medium obtained by mixing two or more kinds of media with each other.

The culture vessel (support) is not particularly limited, and may be, for example, a dish, a well insert, a low adhesive plate, or a plate having a bottom shape such as a U shape, and a V shape. As the culture vessel, for example, a container with a base material (permeable membrane) through which a liquid can pass but cells in the liquid do not pass can also be used. Examples of containers with a permeable membrane include, but are not limited to, cell culture inserts such as a Transwell (registered trademark) insert, a Netwell (registered trademark) insert, a Falcon (registered trademark) cell culture insert, and a Millicell (registered trademark) cell culture insert.

The plurality of cells may be cultured while adhered to the support or the plurality of cells may be cultured without being adhered to the support or may be separated from the support during culture and cultured. In a case where the plurality of cells are cultured without being adhered to the support or in a case where the cells are separated from the support during culture and cultured, it is preferable to use a plate having a bottom shape such as a U shape and a V shape that inhibits adhesion of the cells to the support or a low adsorption plate.

The culturing step in the method for producing a cell structure according to the present embodiment is preferably performed under conditions that allow the medium components to be taken in from all directions of the cell structure. By culturing the plurality of cells or the cell structure under such conditions, the plurality of cells or the cell structure can take in a larger amount of the medium components (mainly growth factors involved in angiogenesis), so that angiogenesis is more promoted in the cell structure. In addition, degeneration of the blood vessels formed in the cell structure can also be suppressed. Here, the plurality of cells form a cell structure along with culturing. In the present specification, the "cell structure" includes not only a cell structure in which blood vessels that do not diffuse to the outside are formed, but also a cell structure at a stage before such blood vessels that do not diffuse to the outside are formed, and a cell structure at an intermediate stage of the formation of blood vessels that do not diffuse to the outside.

A method for culturing cells under conditions that allow medium components to be taken in from all directions of the cell structure includes, for example, a method in which cells or a cell structure are cultured using a container provided with a substrate (permeable membrane) that allows liquid to pass through (for example, the above-described container with an insert), or a method in which cells are embedded in a hydrogel and the gel is immersed in a medium to culture the cells or the cell structure. The embedding of the cells in a hydrogel means that the hydrogel is present on the outside of the cells or on at least a part or the entirety of the outside and the intercellular space. The method for embedding the cells in a hydrogel will be described later. On the other hand, for example, when cells are cultured while being adhered to a dish or a plate, the cells adhered to the bottom surface of the culture vessel cannot take in medium components from the lower side.

The method for producing a cell structure according to the present embodiment may include, prior to the above-described culturing step, a step of obtaining a mixture containing the plurality of cells and an extracellular matrix component (mixing step). In the mixing step, a mixture is obtained by mixing a plurality of cells and extracellular matrix components. By including the mixing step, the plurality of cells are arranged via the extracellular matrix, thereby facilitating the formation of a cell structure in which the plurality of cells are three-dimensionally arranged.

The mixing step may be performed in an aqueous medium. The "aqueous medium" means a liquid that contains water as an essential component. The aqueous medium may be, for example, an aqueous medium including a cationic substance. The aqueous medium including a cationic substance is, for example, a cationic buffer solution such as a tris-hydrochloric acid buffer, a tris-maleic acid buffer, a bis-tris-buffer, or a cationic buffer such as HEPES (4-(2-hydroxyethyl)-1-piperazine ethanesulfonic acid), and the cationic substance may be a medium including a cationic compound such as ethanolamine, diethanolamine, triethanolamine, polyvinylamine, polyallylamine, polylysine, polyhistidine, and polyarginine, and water. In addition, as the aqueous medium, a medium can be used. Examples of mediums include a liquid medium such as a Dulbecco's Modified Eagle medium (DMEM) and a hepatocyte-dedicated medium (HCM). The liquid medium may be a mixed medium in which two types of media have been mixed.

The concentration and the pH of the cationic substance (for example, tris in a tris-hydrochloric acid buffer) in the aqueous medium including a cationic substance are not particularly limited as long as it does not adversely affect cell growth and construction of the cell structure. For example, the concentration of the cationic substance may be from 10 to 100 mM, or from 40 to 70 mM, based on the total volume of the aqueous medium including the cationic substance, and may be 50 mM. The pH of the aqueous medium (for example, a cationic buffer solution) may be from 6.0 to 8.0, from 6.8 to 7.8, or from 7.2 to 7.6.

In the present specification, the "extracellular matrix component" is an aggregate of extracellular matrix molecules formed by a plurality of extracellular matrix molecules. The extracellular matrix molecule means a substance that is present outside cells in an organism. As the extracellular matrix, any substance can be used as long as it does not adversely affect cell growth and formation of cell aggregates. Specific examples of the extracellular matrix molecule include collagen, elastin, proteoglycan, fibronectin, hyaluronic acid, laminin, vitronectin, tenascin, entactin, fibrillin and cadherin, but the present invention is not limited thereto. The extracellular matrix components may be used alone or may be used in combination.

The extracellular matrix may be a modifier or variant of the above extracellular matrix molecule as long as it does not adversely affect the growth of cells and formation of cell aggregates, and may be a polypeptide such as a chemically synthesized peptide. The extracellular matrix may have a repetition of a sequence represented by Gly-X-Y, which is a characteristic of collagen. Here, Gly represents a glycine residue, and X and Y each independently represent any amino acid residue. A plurality of Gly-X-Y's may be the same as or different from each other. When a repetition of a sequence represented by Gly-X-Y is provided, there are few restrictions on the arrangement of molecular chains, and for example, the function as a scaffolding material during cell culture is further improved. In the extracellular matrix having a repetition of a sequence represented by Gly-X-Y, the proportion of the sequence represented by Gly-X-Y may be 80% or more and preferably 95% or more of the complete amino acid sequence. In addition, the extracellular matrix may be a polypeptide having an RGD sequence. The RGD sequence refers to a sequence represented by Arg-Gly-Asp (arginine residue-glycine residue-aspartic acid residue). For example, when the RGD sequence is provided, cell adhesion is further promoted, which makes it more suitable as a scaffolding material during cell culture. Examples of extracellular matrixes including a sequence represented by Gly-X-Y and an RGD sequence include collagen, fibronectin, vitronectin, laminin, and cadherin.

Examples of collagen include fibrous collagen and non-fibrous collagen. The fibrous collagen refers collagen including collagen fibers as a main component, and specific examples thereof include type I collagen, type II collagen, and type III collagen. Examples of the non-fibrous collagen include type IV collagen.

Examples of proteoglycans include chondroitin sulfate proteoglycan, heparan sulfate proteoglycan, keratan sulfate proteoglycan, and dermatan sulfate proteoglycan, but the present invention is not limited thereto.

The shape of the extracellular matrix component is, for example, a fibrous shape. The fibrous shape means a shape composed of a filamentous extracellular matrix component or a shape composed of filamentous extracellular matrix components cross-linked between molecules. At least some of the extracellular matrix components may be fibrous. The shape of the extracellular matrix component is a shape of a mass of the extracellular matrix components (an aggregate of extracellular matrix components) when observed under a microscope, and the extracellular matrix components preferably have an average diameter and/or a size of an average length, which will be described below. The fibrous extracellular matrix component includes a thin filamentous product (fibrils) formed by agglomeration of a plurality of filamentous extracellular matrix molecules, a filamentous product formed by further agglomeration of fibrils, a product in which these filamentous products have been defibrated, and the like. When the extracellular matrix components having a fibrous shape are contained, the RGD sequence is preserved without being destroyed in the fibrous extracellular matrix components, and the component can function as a scaffolding material for cell adhesion more effectively.

The extracellular matrix component may include at least one selected from the group consisting of collagen, laminin, and fibronectin, and preferably includes collagen. The collagen is preferably fibrous collagen, and more preferably type I collagen. The fibrous collagen may be commercially available collagen, and specific examples thereof include type I collagen derived from porcine skin (commercially available from NH Foods Ltd.).

The extracellular matrix component may be derived from animals. Examples of animal species from which an extracellular matrix component is derived include humans, pigs, and cows, but the present invention is not limited thereto. Regarding the extracellular matrix component, a component derived from one kind of animal may be used, or a component derived from a plurality of kinds of animals may be used in combination.

The extracellular matrix component may include fragmented extracellular matrix components. The "fragmentation" means that an aggregate of extracellular matrix components is made smaller in size. The fragmented extracellular matrix components may include defibrated extracellular matrix components. The defibrated extracellular matrix component is a component obtained by defibrating the above extracellular matrix components by applying a physical force. For example, defibration is performed under conditions in which bonds in the extracellular matrix molecules are not broken.

The fragmented extracellular matrix components can be produced by, for example, a method including a step of fragmenting extracellular matrix components (fragmented step).

A method for fragmenting extracellular matrix components is not particularly limited, and the component may be fragmented by applying a physical force. The extracellular matrix components fragmented by applying a physical force have generally the same molecular structure as that before fragmentation, unlike an enzymatic treatment (the molecular structure is maintained). The method for fragmenting extracellular matrix components may be, for example, a method for finely crushing mass extracellular matrix components. The extracellular matrix components may be fragmented in a solid phase or fragmented in an aqueous medium. For example, the extracellular matrix components may be fragmented by applying a physical force with an ultrasonic homogenizer, a stirring homogenizer, a high-pressure homogenizer or the like. In a case where the stirring homogenizer is used, the extracellular matrix component may be homogenized directly or may be homogenized in an aqueous medium such as saline. In addition, it is possible to obtain fragmented extracellular matrix components having a millimeter size or a nanometer size by adjusting the homogenization time, the number of homogenizations, and the like. In a case where the extracellular matrix components are fragmented in an aqueous medium, the fragmented extracellular matrix components can be produced, for example, by a method including a step of fragmenting extracellular matrix components in an aqueous medium and a step of removing an aqueous medium from a liquid containing the fragmented extracellular matrix components and the aqueous medium (removal step). The removal step may be performed by, for example, a freeze-drying method. "Removing an aqueous medium" does not mean that no water is attached to the fragmented extracellular matrix components but means that there is less water than the extent that can be achieved by the above general drying method in common sense.

The diameter and length of the fragmented extracellular matrix components can be determined by analyzing individual fragmented extracellular matrix components using an electron microscope.

The average length of the fragmented extracellular matrix components may be 100 nm or more and 400 µm or less or 100 nm or more and 200 µm or less. In one embodiment, the average length of the fragmented extracellular matrix components may be 5 µm or more and 400 µm or less, 10 µm or more and 400 µm or less, or 100 µm or more and 400 µm or less in order to easily form a thick cell structure. In another embodiment, the average length of the fragmented extracellular matrix components may be 100 µm or less, 50 µm or less, 30 µm or less, 15 µm or less, 10 µm or less, or 1 µm or less, and may be 100 nm or more. The average length of most of the fragmented extracellular matrix components among all the fragmented extracellular matrix components is preferably within the above numerical value range. Specifically, preferably, the average length of 50% or more of the fragmented extracellular matrix components among all the fragmented extracellular matrix components is within the above numerical value range, and more preferably, the average length of 95% of the fragmented extracellular matrix components is within the above numerical value range. The fragmented extracellular matrix components are preferably fragmented collagen components having an average length within the above range.

The average diameter of the fragmented extracellular matrix components may be 50 nm to 30 µm, 4 µm to 30 µm, or 5 µm to 30 µm. The fragmented extracellular matrix components are preferably fragmented collagen components having an average diameter within the above range.

The average length and average diameter of the fragmented extracellular matrix components can be determined by measuring each fragmented extracellular matrix component using an optical microscope and performing image analysis. In this specification, the "average length" means an average value of the lengths of the measured samples in the longitudinal direction, and the "average diameter" means an average value of lengths of the measured samples in a direction orthogonal to the longitudinal direction.

The collagen components that are fragmented are also referred to as "fragmented collagen components." The "fragmented collagen components" means components which are obtained by fragmenting collagen components such as fibrous collagen components, and which maintain a triple helix structure. The average length of the fragmented collagen components is preferably 100 nm to 200 µm, more preferably 22 µm to 200 µm, and still more preferably 100 µm to 200 µm. The average diameter of the fragmented collagen components is preferably 50 nm to 30 µm, more preferably 4 µm to 30 µm, and still more preferably 20 µm to 30 µm.

At least some of the extracellular matrix components may be cross-linked between intermolecularly or intramolecularly. The extracellular matrix components may be cross-linked within extracellular matrix molecules or between extracellular matrix molecules constituting the extracellular matrix components. In a case where the extracellular matrix components include fragmented extracellular matrix components, at least some of the fragmented extracellular matrix components may be cross-linked between intermolecularly or intramolecularly.

The extracellular matrix components of which at least some are cross-linked between intermolecularly or intramolecularly can be produced by, for example, a method including a step of cross-linking extracellular matrix components (cross-linking step). The extracellular matrix components can include, for example, fragmented and cross-linked extracellular matrix components. The fragmented and cross-linked extracellular matrix components can be produced by, for example, a method including a step of fragmenting extracellular matrix components and a step of cross-linking the fragmented extracellular matrix components in that order, or a method including a step of cross-linking extracellular matrix components and a step of fragmenting the cross-linked extracellular matrix components in that order.

Examples of cross-linking methods include a physical cross-linking method by applying heat, ultraviolet rays, radiation, and the like, and a chemical cross-linking method using a cross-linking agent, an enzyme reaction and the like, and the method is not particularly limited. Physical cross-linking is preferable so that cell growth is not hindered. Cross-linking (physical cross-linking and chemical cross-linking) may be cross-linking via covalent bonds.

In a case where the extracellular matrix components include collagen components, cross-links may be formed between collagen molecules (triple helix structure), and may be formed between collagen fibrils formed by collagen molecules. Cross-linking may be cross-linking by heat (thermal cross-linking). Thermal cross-linking can be performed, for example, by performing a heat treatment under a reduced pressure using a vacuum pump. In a case where the collagen components are thermally cross-linked, the extracellular matrix components may be cross-linked when amino groups of collagen molecules form peptide bonds (-NH-CO-) with carboxyl groups of the same or other collagen molecules.

The extracellular matrix components can be cross-linked using a cross-linking agent. The cross-linking agent can, for example, cross-link carboxyl groups and amino groups, or can cross-link amino groups with each other. As the cross-linking agent, for example, an aldehyde-based, carbodiimide-based, epoxide-based or imidazole-based cross-linking agent is preferable in consideration of cost, safety and operability, and specific examples thereof include water-soluble carbodiimides such as glutaraldehyde, 1-ethyl-3-(3-dimethylaminopropyl)carbodiimide/hydrochloride, and 1-cyclohexyl-3-(2-morpholinyl-4-ethyl)carbodiimide/sulfonate.

The degree of cross-linking can be appropriately selected according to the type of the extracellular matrix components, a method for cross-linking, and the like. The degree of cross-linking may be 1% or more, 2% or more, 4% or more, 8% or more, or 12% or more, and may be 30% or less, 20% or less, or 15% or less. When the degree of cross-linking is within the above range, the extracellular matrix molecule can be appropriately dispersed, and the redispersibility after dry storage is favorable.

In a case where amino groups in the extracellular matrix components are used for cross-linking, the degree of cross-linking can be quantified based on a TNBS (2,4,6-trinitrobenzenesulfonic acid) method described in Acta Biomaterialia, 2015, vol. 25, pp. 131-142. The degree of cross-linking by the TNBS method may be within the above range. The degree of cross-linking by the TNBS method is a proportion of amino groups used for cross-linking among amino groups of the extracellular matrix. In a case where the extracellular matrix components include collagen components, the degree of cross-linking measured by the TNBS method is preferably within the above range.

The degree of cross-linking may be calculated by quantifying carboxyl groups. For example, in the case of extracellular matrix components that are insoluble in water, quantification may be performed by a TBO (toluidine blue O) method. The degree of cross-linking by the TBO method may be within the above range.

In the step of cross-linking, the temperature (heating temperature) and the time (heating time) when the extracellular matrix components are heated can be appropriately determined. The heating temperature may be, for example, 100°C or higher, 200°C or lower, or 220°C or lower. The heating temperature may be, specifically, for example, 100°C, 110°C, 120°C, 130°C, 140°C, 150°C, 160°C, 170°C, 180°C, 190°C, 200°C, or 220°C. The heating time (the time during which the heating temperature is maintained) can be appropriately set according to the heating temperature. In a case where heating is performed at, for example, 100°C to 200°C, the heating time may be 6 hours or more and 72 hours or less, and is more preferably 24 hours or more and 48 hours or less. In the step of cross-linking, heating may be performed in the absence of a solvent or heating may be performed under a reduced pressure condition.

The content of the extracellular matrix components in the mixture in the mixing step can be appropriately determined depending on the shape, thickness, and the like of a target cell structure. The content of the extracellular matrix components in the mixture may be, for example, 0.005 mg/mL or more, 0.01 mg/mL or more, 0.025 mg/mL or more, 0.05 mg/mL or more, 0.10 mg/mL or more, 0.15 mg/mL or more, 0.20 mg/mL or more, 0.25 mg/mL or more, 0.30 mg/mL or more, 0.35 mg/mL or more, 0.40 mg/mL or more, or 0.45 mg/mL or more, and 1.5 mg/mL or less, 1.25 mg/mL or less, 1.0 mg/mL or less, 0.8 mg/mL or less, or 0.6 mg/mL or less, based on the total amount of the mixture.

The mixture in the mixing step may further contain a polymer electrolyte. The polymer electrolyte is a high-molecular-weight compound having properties of an electrolyte. Examples of the polymer electrolyte include, but are not limited to, glycosaminoglycans such as heparin, chondroitin sulfate (for example, chondroitin 4-sulfate and chondroitin 6-sulfate), heparan sulfate, dermatan sulfate, keratan sulfate, hyaluronic acid; and dextran sulfate, rhamnan sulfate, fucoidan, carrageenan, polystyrene sulfonic acid, polyacrylamide-2-methylpropanesulfonic acid, polyacrylic acid, and derivatives thereof. The polymer electrolytes may consist of one described above or may contain a combination of two or more thereof.

The polymer electrolyte is preferably a glycosaminoglycan, more preferably includes at least one selected from the group consisting of heparin, dextran sulfate, chondroitin sulfate, and dermatan sulfate, and even more preferably includes heparin. In a case where the mixture in the mixing step contains a polymer electrolyte, excessive aggregation of the extracellular matrix components can be more effectively suppressed, and as a result, it becomes easier to obtain a cell structure exhibiting excellent responsiveness to a given substance. In a case where the cell structure contains heparin as the polymer electrolyte, the effect becomes even more pronounced.

The concentration of the polymer electrolyte in the mixture in the mixing step is not particularly limited as long as it does not adversely affect the growth of cells and the formation of the cell structure. The concentration of the polymer electrolyte in the mixture may be more than 0 mg/mL and 1.5 mg/mL or less based on the total amount of the mixture. The concentration of the polymer electrolyte in the mixture may be 0.005 mg/mL or more, 0.01 mg/mL or more, 0.02 mg/mL or more, 0.03 mg/mL or more, or 0.04 mg/mL or more, and may be 1.5 mg/mL or less, 1.0 mg/mL or less, 0.1 mg/mL or less, 0.08 mg/mL or less, or 0.06 mg/mL or less. The concentration of the polymer electrolyte in the mixture may be, for example, 0.025 mg/mL, 0.05 mg/mL, 0.075 mg/mL, or 0.1 mg/mL.

The ratio (C2/C1) of the mass C2 of the polymer electrolyte to the mass C1 of the extracellular matrix components may be 1/100 to 100/1, 1/10 to 10/1, 1/5 to 5/1 or 1/2 to 2/1, and may be 1/1.5 to 1.5/1.

The mixture in the mixing step may further contain a hydrogel precursor, and in such a case, the method for producing a cell structure according to the present embodiment may include, after the mixing step and before the culturing step, a step of gelling the hydrogel precursor in the mixture (gelation step).

The gelation of the mixture containing the hydrogel precursor may include incubating the mixture for a certain period of time.

A hydrogel precursor is a substance that forms a hydrogel in response to an external stimulus such as a chemical or physical stimulus, and is a substance in a state in which a hydrogel has not yet been formed. Examples of hydrogels include fibrin gel, collagen gel, gelatin gel, hyaluronic acid gel, alginic acid gel, and pectin gel. Examples of hydrogel precursors include, for example, fibrinogen and thrombin, collagen, gelatin, hyaluronic acid, alginic acid, and pectin. As the hydrogel precursor, it is preferable to include fibrinogen and thrombin. Fibrin is a component produced by the action of thrombin on fibrinogen to release Aα chains, A chains from the N terminal of Bβ chains, and B chains. Fibrin is formed by bringing fibrinogen into contact with thrombin.

The concentration of the hydrogel precursor in the mixture in the mixing step may be, for example, 0.1 mg/mL or more, 0.2 mg/mL or more, 0.3 mg/mL or more, 0.4 mg/mL or more, 0.5 mg/mL or more, 0.6 mg/mL or more, 0.7 mg/mL or more, 0.8 mg/mL or more, 0.9 mg/mL or more, or 1.0 mg/mL or more, based on the total amount of the mixture. The concentration of the hydrogel precursor in the mixture in the mixing step may be 10.0 mg/mL or less, 8.0 mg/mL or less, 6.0 mg/mL or less, 5.0 mg/mL or less, 3.0 mg/mL or less, 1.0 mg/mL or less, 0.8 mg/mL or less, or 0.6 mg/mL or less, based on the total amount of the mixture.

For example, in a case where the hydrogel precursor includes two or more substances, such as a combination of fibrinogen and thrombin, a mixture containing cells and the hydrogel precursor may be obtained by mixing a first composition containing cells and a first hydrogel precursor (for example, thrombin) with a second composition containing a second hydrogel precursor (for example, fibrinogen). The second composition may contain cells, and both the first composition and the second composition may contain cells.

### [Method for Forming Blood Vessel that do not Diffuse to Outside in Cell Structure]

As described above, the production method according to the present embodiment enables the production of a cell structure which includes epithelial cells and in which blood vessels that do not diffuse to the outside are formed. Accordingly, the present invention can also be understood as a method for forming blood vessels that do not diffuse to the outside in a cell structure including epithelial cells, the method including culturing a plurality of cells including at least epithelial cells and vascular endothelial cells in a medium including one or more growth factors involved in angiogenesis. Specific aspects of the method are not particularly limited, and each of the above-described aspects can be applied as appropriate.

### [Cell Structure]

The cell structure according to the present embodiment includes a plurality of cells including epithelial cells and vascular endothelial cells, and blood vessels that do not diffuse to the outside are formed. The cell structure according to the present embodiment may include an extracellular matrix component, and may further include a polymer electrolyte. The cell structure according to the present embodiment can be obtained by, for example, the above-described method.

In a case where the cell structure according to the present embodiment includes the extracellular matrix component, the content of the extracellular matrix component in the cell structure may be, for example, 0.01% by mass or more, 0.05% by mass or more, 0.1% by mass or more, 0.5% by mass or more, 1% by mass or more, 2% by mass or more, 3% by mass or more, 4% by mass or more, 5% by mass or more, 6% by mass or more, 7% by mass or more, 8% by mass or more, 9% by mass or more, 10% by mass or more, 15% by mass or more, 20% by mass or more, 25% by mass or more, or 30% by mass or more, and 90% by mass or less, 80% by mass or less, 70% by mass or less, 60% by mass or less, 50% by mass or less, 30% by mass or less, 20% by mass or less, or 15% by mass or less, based on the dry weight of the cell structure. The content of the extracellular matrix component in the cell structure may be from 0.01% to 90% by mass, from 10% to 90% by mass, from 10% to 80% by mass, from 10% to 70% by mass, from 10% to 60% by mass, from 1% to 50% by mass, from 10% to 50% by mass, from 10% to 30% by mass, or from 20% to 30% by mass, based on the dry weight of the cell structure.

The blood vessels formed in the cell structure according to the present embodiment may have further suppression of degeneration due to the growth factors involved in angiogenesis described above.

In the cell structure according to the present embodiment, the average length of blood vessels formed in the cell structure may be, for example, 500 µm or more, 600 µm or more, 700 µm or more, or 800 µm or more. In addition, the average length of blood vessels formed in the cell structure may be 3000 µm or less, 2500 µm or less, 2000 µm or less, or 1500 µm or less.

In the cell structure according to the present embodiment, in a case where the epithelial cells are hepatocytes, expression of a metabolic enzyme gene belonging to the cytochrome P450 superfamily specific to hepatocytes, or activity of the metabolic enzyme may be sufficiently maintained. Examples of metabolic enzymes belonging to the cytochrome P450 superfamily include CYP3A4, CYP2C9, CYP1A1, CYP1A2, CYP2E1, and CYP27A1. Since CYP3A4 is an enzyme involved in the metabolism of a large number of compounds, it is particularly important that its expression and activity are maintained during the culture period. In the cell structure according to the present embodiment, in a case where the epithelial cells are hepatocytes, the activity of the CYP3A4 enzyme may be particularly well maintained.

In the cell structure according to the present embodiment, in a case where the epithelial cells are hepatocytes, the gene expression level of the metabolic enzyme may be 0.6 times or more, 0.65 times or more, 0.7 times or more, 0.75 times or more, or 0.8 times or more, and may be 2 times or less, 1.9 times or less, 1.8 times or less, 1.7 times or less, 1.6 times or less, 1.5 times or less, 1.4 times or less, or 1.3 times or less, based on the activity of the metabolic enzyme gene after the production of the cell structure (for example, four days after the start of cell culturing).

The thickness of the cell structure according to the present embodiment may be 10 µm or more, 30 µm or more, 50 µm or more, 100 µm or more, 300 µm or more, or 1,000 µm or more. Such a cell structure is a structure closer to that of a living tissue, and is suitable as a substitute for an experimental animal and a material for transplantation. The upper limit of the thickness of the three-dimensional hepatic tissue is not particularly limited, and may be, for example, 10 mm or less, 3 mm or less, 2 mm or less, 1.5 mm or less, 1 mm or less, 300 µm or less, 200 µm or less, 150 µm or less, or 100 µm or less.

Here, the "thickness of the cell structure" means a distance between both ends in a direction perpendicular to the main surface in a case where the cell structure is sheet-like or a rectangular parallelepiped. In a case where the main surface is uneven, the thickness means a distance at the thinnest part of the main surface.

In a case where the cell structure is spherical or substantially spherical, the thickness of the cell structure means the diameter of the cell structure. In a case where the cell structure means ellipsoidal or substantially ellipsoidal, the thickness of the cell structure is the breadth of the cell structure. In a case where the cell structure is substantially spherical or substantially ellipsoidal, and the surface is uneven, the thickness of the cell structure is a distance between two points where the straight line passing through the center of gravity of the cell structure and the surface intersect, and means the shortest distance.

### [Example]

Hereinafter, the present invention will be described in more detail based on examples. However, the present invention is not limited to these examples.

### [Test Example 1: Confirmation of Effect on Angiogenesis by Growth Factors Involved in Angiogenesis]

A cell mixture of 5.85 × 10⁵ human hepatocytes (PXB Cells (registered trademark); manufactured by PhoenixBio Co., Ltd.) and 2.25 × 105 vascular endothelial cells (GFP-HUVEC; manufactured by ANGIO-PROTEOMIE) was suspended in an equal-volume mixed solution (heparin-collagen solution) of 50 µL of 1.0 mg/mL heparin/200 mM Tris-hydrochloric acid buffer (pH 7.4) and 50 µL of 0.6 mg/mL collagen/5 mM acetic acid solution (pH 3.7). The obtained mixture was centrifuged at 400 × g for 2 minutes at room temperature, the supernatant was removed, and the cells were resuspended in HCM medium (Product No. "CC-3198"; manufactured by Lonza) containing 10 U/mL of thrombin (Product No. "R4648"; manufactured by Sigma-Aldrich) and 1 v/v% endothelial cell growth supplement (ECGS) (Product No. "1052"; manufactured by Sciencell) to a cell density of 2.7 × 10⁴ cells/2 µL, thereby obtaining a cell suspension. In the cell suspension, the cell density of human hepatocytes was set to 1.95 × 10⁴ cells/2 µL, and the cell density of vascular endothelial cells was set to 7.5×10³ cells/2 µL.

A droplet was formed by adding 2 µL of an HCM medium including 5 mg/mL of fibrinogen and 1 v/v% ECGS onto a 48-well microplate (Product No. "3830-048"; manufactured by AGC TECHNO GLASS Co., Ltd.). Subsequently, the above cell suspension was added into the droplet, and the 48-well microplate was then left to stand in an incubator for 40 minutes to form a fibrin gel in which the human hepatocytes and vascular endothelial cells were embedded. For each well in which the fibrin gel was formed, 0.5 mL of an HCM medium including 50 ng/mL VEGF-A as a vascular development factor, or 50 ng/mL VEGF-A as a vascular development factor and 200 ng/mL angiopoietin-1 as a vascular maintenance factor, along with 1 v/v% ECGS, was added, and the culture was carried out under conditions of 37°C and 5% CO₂ to obtain a cell structure. The culture was performed by replacing the medium every two days after the start of the culture. As a control, a cell structure was obtained in the same manner as described above, except that a fibrin gel embedding the human hepatocytes and vascular endothelial cells was formed and the cells were cultured in an HCM medium that did not contain ECGS or growth factors involved in angiogenesis.

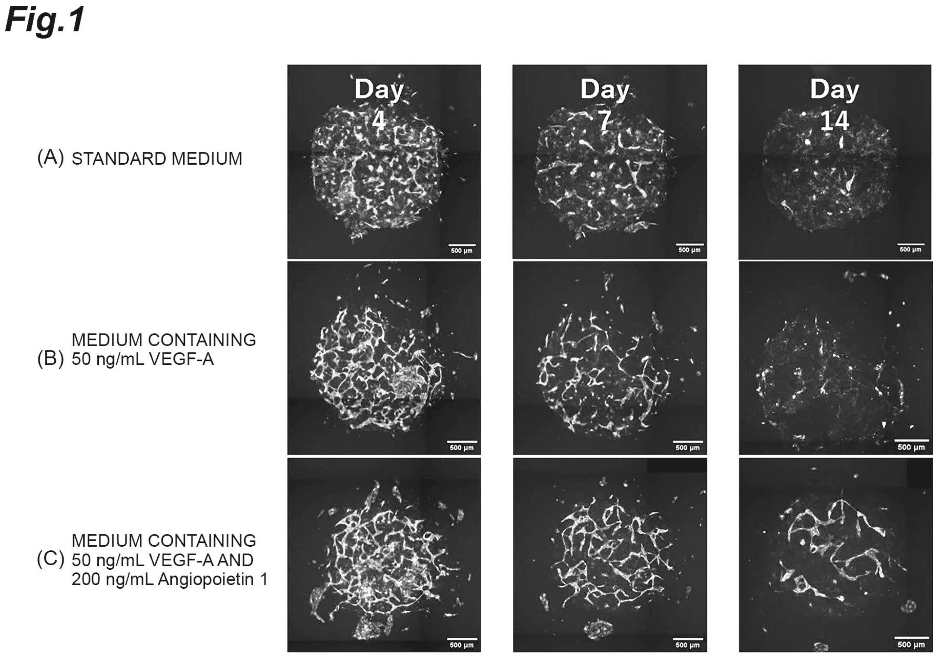

Vascular endothelial cells (GFP-HUVEC) were observed using a confocal microscope on the obtained cell structure, and angiogenesis in the cell structure was evaluated. The results are illustrated in FIG. 1.

As illustrated in FIG. 1, the cell structures cultured in the medium including VEGF-A had formed blood vessels and no diffusion of the blood vessels to the outside was observed (FIG. 1(B)). In addition, the cell structure cultured in a medium including VEGF-A and angiopoietin-1 exhibited further enhanced angiogenesis. Furthermore, in the cell structure cultured in a medium containing VEGF-A and angiopoietin-1, degeneration of the blood vessels was alleviated even on Day 14 of culturing, as compared with the cell structure cultured in a medium containing VEGF-A, and no diffusion of the blood vessels to the outside was observed (FIG. 1(C)). In the cell structure cultured in a standard medium that did not contain the growth factors involved in angiogenesis, no angiogenesis was observed (FIG. 1(A)).

### [Test Example 2: Confirmation of Effect of Culture Method on Angiogenesis]

Except for using a 48-well microplate or a 24-transwell (manufactured by Corning Incorporated) as the culture vessel, fibrin gels in which human hepatocytes and vascular endothelial cells were embedded were formed in the same manner as in Test Example 1. A composition containing insulin, hemopexin, EGF, melanotransferrin, IGF-1, LRG1, and HSPG2, which are vascular maintenance factors (hereinafter also referred to as "vascular maintenance factor cocktail"), was prepared. Except for using the medium including 50 ng/mL VEGF-A as a vascular development factor and 1 v/v% the vascular maintenance factor cocktail, or the medium including 50 ng/mL VEGF-A and 50 ng/mL VEGF-C as vascular development factors and 1 v/v% the vascular maintenance factor cocktail, the cell structures were obtained by culturing the cells in the same manner as in Test Example 1.

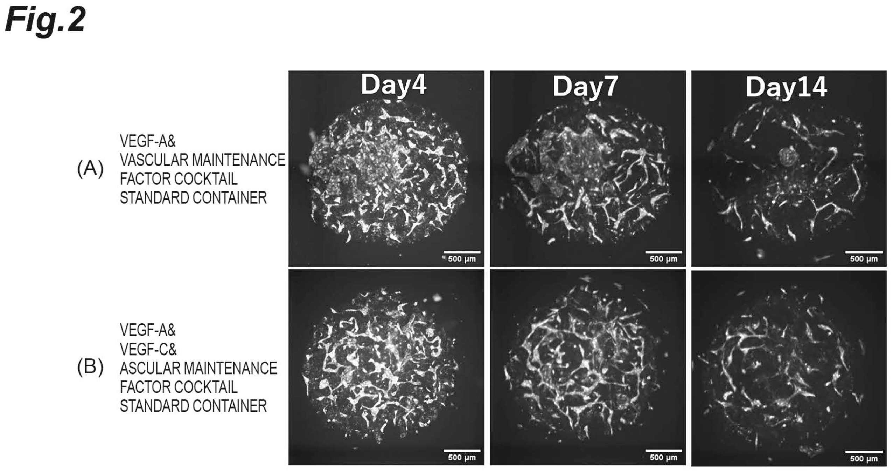

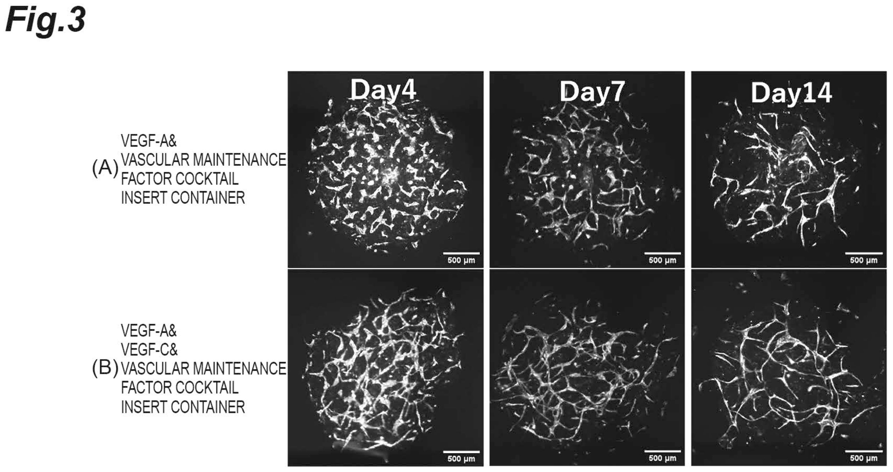

First, with respect to the obtained cell structures, blood vessel formation was evaluated by observing vascular endothelial cells (GFP-HUVEC) under a microscope, in the same manner as in Test Example 1. The results are shown in FIGS. 2 and 3.

As illustrated in FIGS. 2 and 3, compared with the cell structures cultured and produced using a 48-well microplate, the cell structures cultured and produced using a container with an insert such as a 24-transwell under conditions that allow uptake of medium components from all directions of the cells exhibited further suppression of degeneration of the blood vessels (Comparison between FIG. 2(A) and FIG. 3(A), or between FIG. 2(B) and FIG. 3(B)).

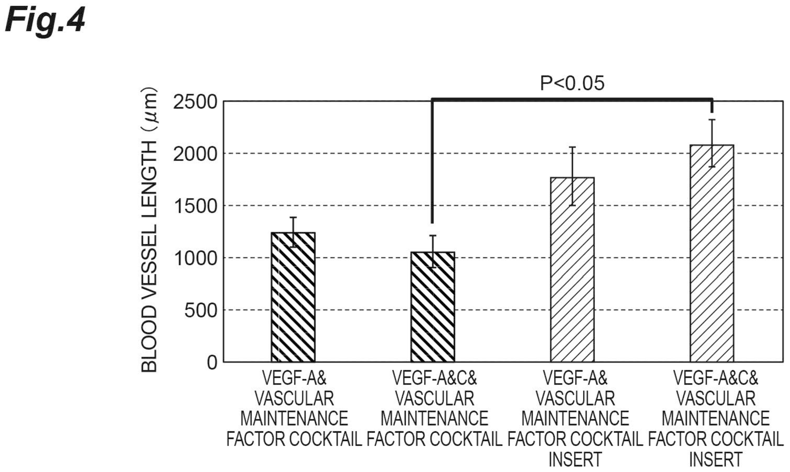

Next, the blood vessel length of the cell structure was measured using Image J based on the microscopic images of the cell structure on Day 14 of culture (number of cell structure samples: 6). The results are illustrated in FIG. 4. As illustrated in FIG. 4, the blood vessel length in the cell structures cultured using a 24-transwell was significantly longer than that in the cell structures cultured using a 48-well microplate. From this result, it was found that by using a container equipped with a permeable membrane or the like and culturing cells under conditions that allow uptake of medium components from all directions of the cells, degeneration of the blood vessels is further suppressed.

### [Test Example 3: Confirmation of Effect of Growth Factors Involved in Angiogenesis on Metabolism-Related Gene Expression]

Except for using media containing 50 ng/mL VEGF-A and 200 ng/mL angiopoietin-1 (ANG), 50 ng/mL VEGF-A and 1 v/v% vascular maintenance factor cocktail, 50 ng/mL VEGF-A alone, 50 ng/mL VEGF-C and 200 ng/mL angiopoietin-1, or media not containing growth factors involved in angiogenesis, the cells were cultured in the same manner as in Test Example 1 to obtain cell structures.

In addition, except for using a 24-transwell as the culture vessel and media containing 50 ng/mL VEGF-A and 200 ng/mL angiopoietin-1 (ANG), 50 ng/mL VEGF-A and 1 v/v% vascular maintenance factor cocktail, or 50 ng/mL VEGF-A, 50 ng/mL VEGF-C and 1 v/v% vascular maintenance factor cocktail, the cells were cultured in the same manner as in Test Example 2 to obtain cell structures.

Except for using a cell mixture of 5.85 × 10⁵ human hepatocytes, 2.25 × 10⁵ vascular endothelial cells (HUVEC), and 9.0 × 10⁴ hepatic stellate cells (LX2; manufactured by Sigma-Aldrich), which was resuspended to a cell density of 3 × 10⁴ cells/2 µL to obtain a cell suspension, cell structures containing hepatic stellate cells were obtained in the same manner as in Test Example 1.

RNA was extracted from the cell structures on Day 1, Day 4, and Day 8 of culturing, and the expression levels of the CYP3A4 gene and the CYP2C9 gene in each of the cell structures were analyzed. For RNA extraction, a Direct-zol RNA Kit (Product No. "R2061", manufactured by Zymo Research) was used. Reverse transcription was performed on the extracted RNA to synthesize complementary DNA (cDNA). For cDNA synthesis, the SuperScript (registered trademark) VILO (registered trademark) cDNA Synthesis Kit (Product No. "11754050"; manufactured by Thermo Fisher) was used. Quantitative PCR analysis was performed on the synthesized cDNA to analyze the expression levels of CYP3A4, CYP2C9, and GAPDH. Quantitative PCR analysis was performed using Taqman (registered trademark) Fast Advanced Master Mix (Product No. "4444556"; manufactured by Thermo Fisher) and Taqman (registered trademark) Gene Expression assay (Product No. "4331182"; manufactured by Thermo Fisher) were used. As primers for the quantitative PCR, oligonucleotides with Assay IDs Hs00604506_m1 for CYP3A4, Hs00426397_m1 for CYP2C9, and Hs99999905_m1 for GAPDH were used. The analysis was performed using the StepOnePlus Real-Time PCR System (manufactured by Thermo Fisher) following the manufacturer's recommended thermal profile.

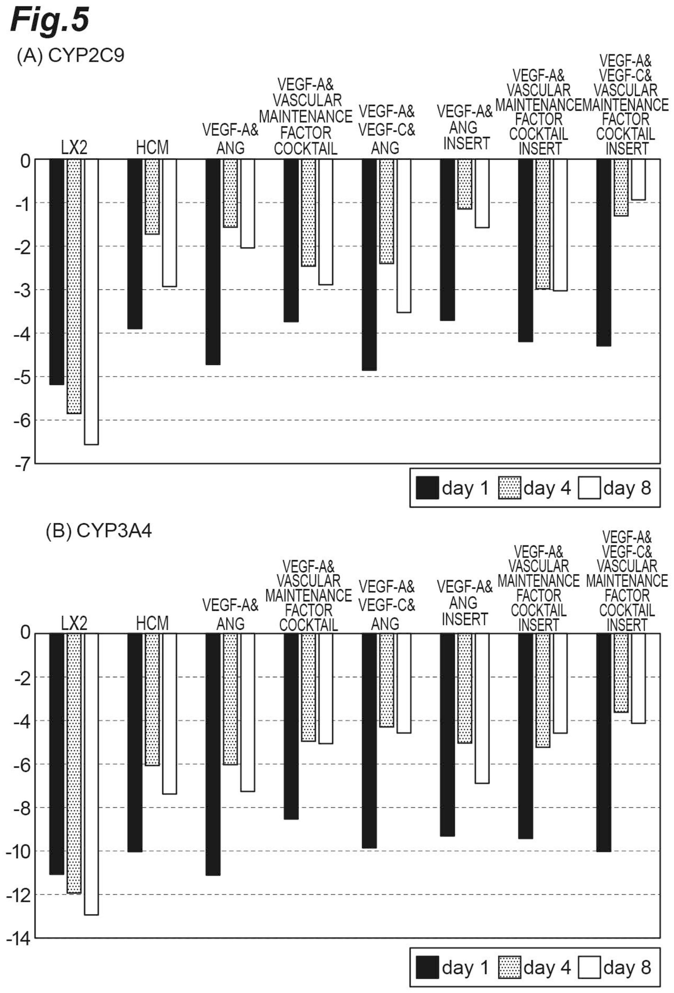

FIG. 5 is a graph illustrating the results of the analysis of the expression levels of the CYP3A4 gene (FIG. 5(A)) and the CYP2C9 gene (FIG. 5(B)). The vertical axis in FIG. 5 shows the relative values of the Ct value (ΔCt) of CYP3A4 or CYP2C9 to the amplification cycle number (Ct value) of GAPDH.

As illustrated in FIG. 5, there were no differences in metabolic-related gene expression between media conditions. Thus, no direct adverse effects on liver function were observed by the growth factors involved in each angiogenesis. In addition, it was shown that the expression of metabolism-related genes gradually decreased during culture in the cell structures containing hepatic stellate cells, indicating difficulty in maintaining liver function (bar graph for LX2 in FIG. 5). On the other hand, cell structures that did not contain hepatic stellate cells did not show a decrease in the expression of metabolism-related genes, indicating that liver function was maintained.

### [Test Example 4: Confirmation of Effect of Different Types of Vascular Endothelial Cells on Angiogenesis]

Except for using a 24-transwell as the culture vessel and using immortalized sinusoidal endothelial cells (SEC), derived from Sciencell's Product No. 5000 and immortalized by introduction of hTERT and SV40LT, instead of HUVECs as vascular endothelial cells, cell structures were obtained in the same manner as in Test Example 2.

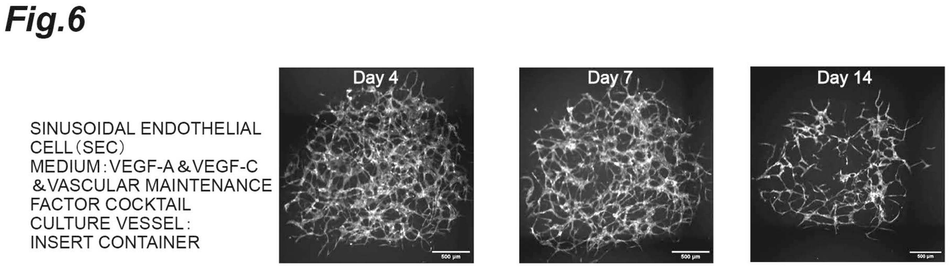

Since the SEC used in Test Example 4 expresses GFP, the SEC was observed in the cell structures produced using SEC as vascular endothelial cells, and blood vessel formation was evaluated in the same manner as in Test Example 2. The results are illustrated in FIG. 6.

Referring also to the results of Test Example 2, angiogenesis was more enhanced in cell structures produced using SECs compared to cell structures produced using HUVECs as vascular endothelial cells (see FIGS. 3 and 6). In addition, no diffusion of blood vessels to the outside was observed in the cell structures produced using SEC (FIG. 6).

Next, as in Test Example 2, the blood vessel length of the cell structures produced using SEC was measured from microscopic images on Day 14 of culturing (number of cell structure samples: 6). The results are shown in Table 1. Referring also to the results of Test Example 2, the blood vessel length in the cell structures produced using SEC was significantly longer than that in the cell structures produced using HUVEC (see FIG. 4 and Table 1).

**[Table 1]**

| Cell structure sample | 1 | 2 | 3 | 4 | 5 | 6 | Average |
|---|---|---|---|---|---|---|---|
| Blood vessel length | 2491.10 | 5140.74 | 4270.45 | 3793.31 | 5063.47 | 4758.91 | 4253.00 |

### [Reference Example: Confirmation of Effect of Hepatic Stellate Cell Secretions on Angiogenesis]

Using the same method as the control in Test Example 1, human hepatocytes and HUVECs were cultured for a predetermined period to obtain a cell structure 1 composed of human hepatocytes and HUVECs. The cell structure 1 is a cell structure at a stage prior to the formation of blood vessels that do not diffuse to the outside. In addition, except for preparing a cell suspension with hepatic stellate cells (LX2) at a cell density of 3 × 10⁴ cells/2 µL instead of human hepatocytes and HUVECs, the cell structure 2 composed only of hepatic stellate cells was obtained by the same method as the control in Test Example 1. Subsequently, the obtained cell structures 1 and 2 were further cultured together in the same well for a predetermined period.

The cell structure 1 cultured in the same well as the cell structure 2 was observed under a microscope and evaluated for blood vessel formation in the same manner as in Test Example 1. The results are illustrated in FIG. 7.

As illustrated in FIG. 7, culturing cell structure 1 together with cell structure 2 in the same well, that is, culturing the cell structure 1 in a medium including secretions from hepatic stellate cells resulted in blood vessel formation. On the other hand, blood vessels observed on the cell structure (within the dashed area in FIG. 7) were confirmed to diffuse outside the cell structure over the course of culture, and thus, a cell structure in which blood vessels that do not diffuse to the outside are formed was not obtained.

### [Test Example 5: Confirmation of Effect of Cell Types on Vascular Structure]

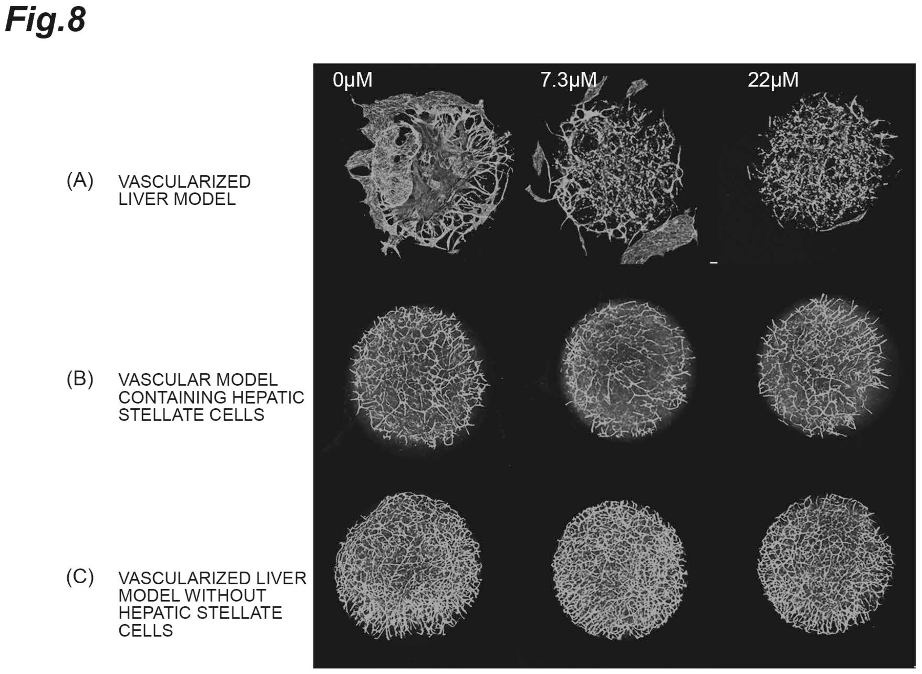

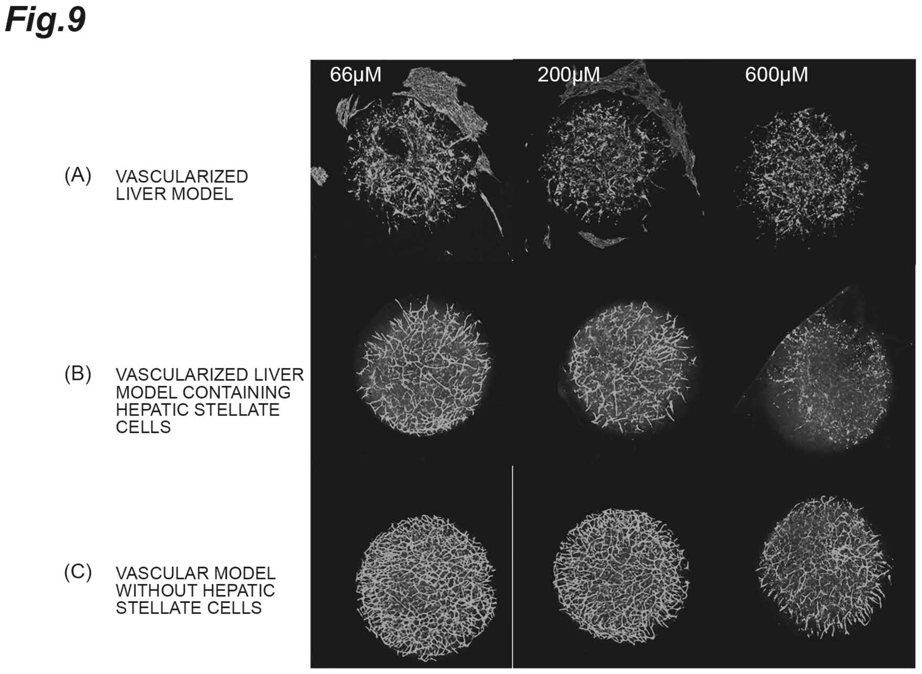

Except for using a 24-transwell as the culture vessel, culturing a cell mixture of 1.95 × 10⁴ human hepatocytes (PXB Cells (registered trademark); manufactured by PhoenixBio Co., Ltd.) and 3.0 × 10³ immortalized sinusoidal endothelial cells (SEC; manufactured by Sciencell, Product No. 5000) using the HCM medium including 50 ng/mL VEGF-A and 50 ng/mL VEGF-C as vascular development factors and 1 v/v% vascular maintenance factor cocktail,culturing a cell mixture of human hepatocytes (PXB Cells (registered trademark); manufactured by PhoenixBio Co., Ltd.), 7.5 × 10³ immortalized sinusoidal endothelial cells (SEC, manufactured by Sciencell, Product No. "5000"), and 3.0 × 10³ hepatic stellate cells (LX2; manufactured by Sigma-Aldrich) using the HCM medium including 1 v/v% vascular maintenance factor cocktail, orculturing a cell mixture of 2.1 × 10³ immortalized sinusoidal endothelial cells (SEC; manufactured by Sciencell, Product No. "5000") and 9.0 × 10³ hepatic stellate cells (LX2; manufactured by Sigma-Aldrich) using the HCM medium including 1 v/v% vascular maintenance factor cocktail, cell mixtures were cultured and cell structures were obtained in the same manner as in Test Example 4. On Day 7 of the cell structure culture, monoclothrin was added to the medium at concentrations of 0 µM, 7.3 µM, 22 µM, 66 µM, 200 µM, or 600 µM, and the cell structures were further cultured in the respective media for an additional 7 days. Thereafter, the cell structures were fixed with 4% paraformaldehyde (PFA), subjected to immunostaining for CD31 according to a standard method, and the vascular structures were observed using a confocal microscope. The results are illustrated in FIGS. 8 and 9.

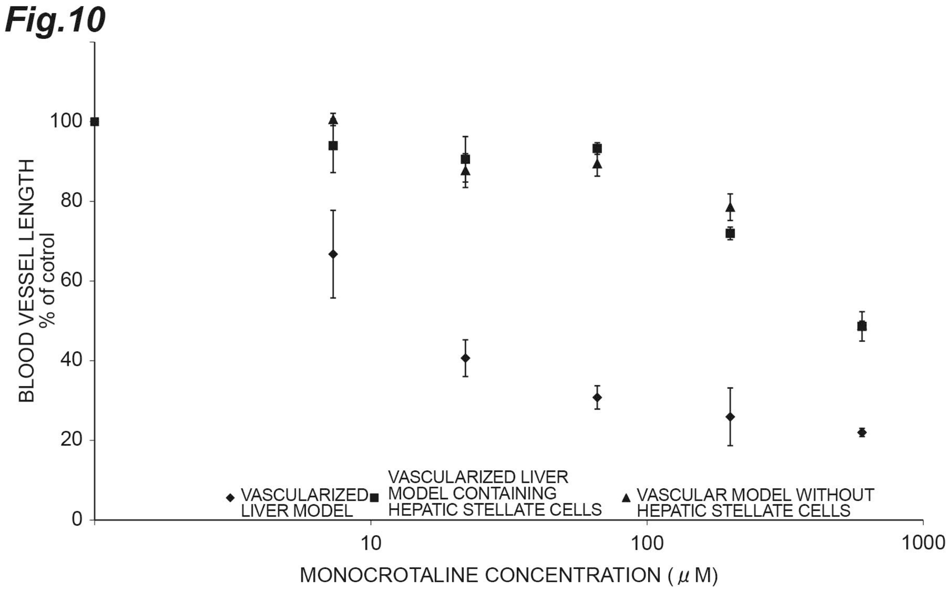

In addition, vascular structures were extracted from the microscopic images shown in FIGS. 8 and 9, and the blood vessel lengths of the cell structures were measured. The results are illustrated in FIG. 10. In FIG. 10, the blood vessel length in each cell structure is shown as a percentage relative to the blood vessel length when monocrotaline was not added to the medium, which is set as 100%. Hereinafter, the cell structure produced using a cell mixture of hepatocytes and immortalized sinusoidal endothelial cells is referred to as a "vascularized liver model", the cell structure produced using a cell mixture of hepatocytes, immortalized sinusoidal endothelial cells, and hepatic stellate cells is referred to as a "vascularized liver model containing hepatic stellate cells", and the cell structure produced using a cell mixture of immortalized sinusoidal endothelial cells and hepatic stellate cells is also referred to as a "vascular model".

Monocrotaline is metabolized by CYP enzymes, which are highly expressed in hepatocytes, into compounds that exhibit vascular toxicity. Even when monocrotaline is administered to models with reduced CYP enzyme activity or models that do not contain hepatocytes, vascular toxicity is not observed.

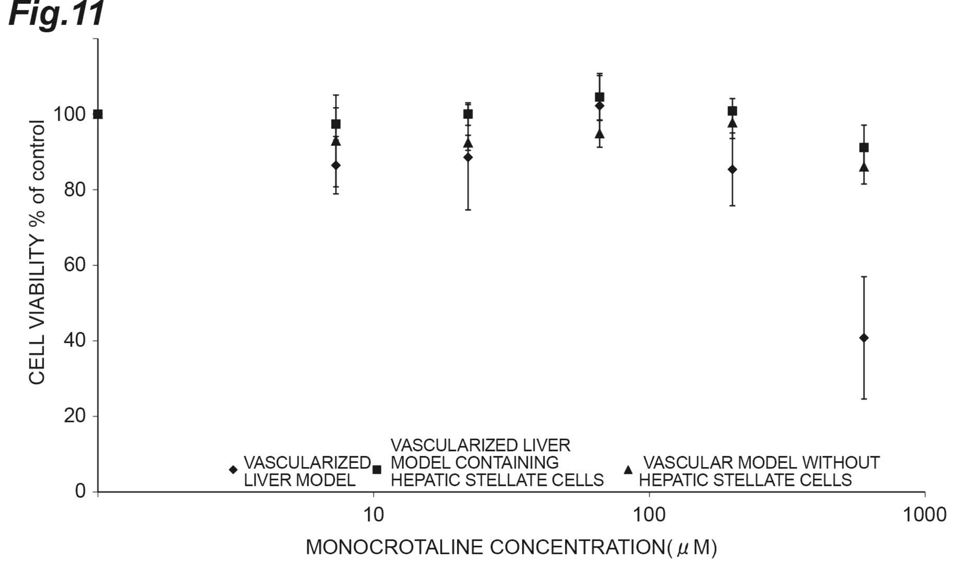

In addition, the cell viability of the similarly produced cell structure was also quantified by an ATP assay. The ATP assay was performed using the CellTiter-Glo (registered trademark) 3D Cell Viability Assay Kit according to the following procedure. From each well of the plate used for culturing each cell structure, the medium including the compound was removed, 100 µL of DMEM at room temperature was added, followed by the addition of 100 µL of the ATP assay reagent provided with the kit at room temperature. The plate to which the ATP assay reagent was added was shaken with a thermostat shaker for 5 minutes (1,000 rpm, room temperature). After that, the plate was left at room temperature for 25 minutes. From each well, the entire volume of the mixed solution of a DMEM and an ATP assay reagent was transferred to a 96 plate for light emission measurement, and the light emission intensity was measured with a plate reader. The results are illustrated in FIG. 11.

As illustrated in FIG. 10, the vascularized liver model exhibited sensitivity to lower concentrations of monocrotaline compared to other models, with a notably shorter blood vessel length. As illustrated in FIG. 11, the vascularized liver model demonstrated a monocrotaline concentration-dependent decrease in cell viability compared to other models. These results suggest that the vascularized liver model maintains higher monocrotaline metabolic capacity and possesses vascular toxicity sensitivity as a liver model.

### [Test Example 6: Quantification of CYP3A4 Enzyme Activity in Cell Structures]

Except for culturing 1.95 × 10⁴ human hepatocytes (PXB Cells (registered trademark); manufactured by PhoenixBio Co., Ltd.) using the HCM medium including 1 v/v% vascular maintenance factor cocktail,culturing a cell mixture of 1.95 × 10⁴ human hepatocytes (PXB Cells (registered trademark); manufactured by PhoenixBio Co., Ltd.) and 3.0 × 10³ immortalized sinusoidal endothelial cells (SEC, manufactured by ScienCell, Product No. "5000") using the HCM medium including 50 ng/mL VEGF-A and 50 ng/mL VEGF-C as vascular development factors, and 1 v/v% vascular maintenance factor cocktail, orculturing a cell mixture of 1.95 × 10⁴ human hepatocytes (PXB Cells (registered trademark); manufactured by PhoenixBio Co., Ltd.), 7.5 × 10³ immortalized sinusoidal endothelial cells (SEC, ScienCell, Product No. "5000"), and 3.0 × 10³ hepatic stellate cells (LX2; manufactured by Sigma-Aldrich) using the HCM medium including 1 v/v% vascular maintenance factor cocktail, the cell mixture was cultured in the same manner as in Test Example 5 to obtain the cell structure. Hereinafter, the cell structure prepared using human hepatocytes is referred to as the "liver model," the cell structure prepared using human hepatocytes and immortalized sinusoidal endothelial cells is referred to as the "vascularized liver model," and the cell structure prepared using human hepatocytes, immortalized sinusoidal endothelial cells, and hepatic stellate cells is also referred to as the "vascularized liver model containing hepatic stellate cells".

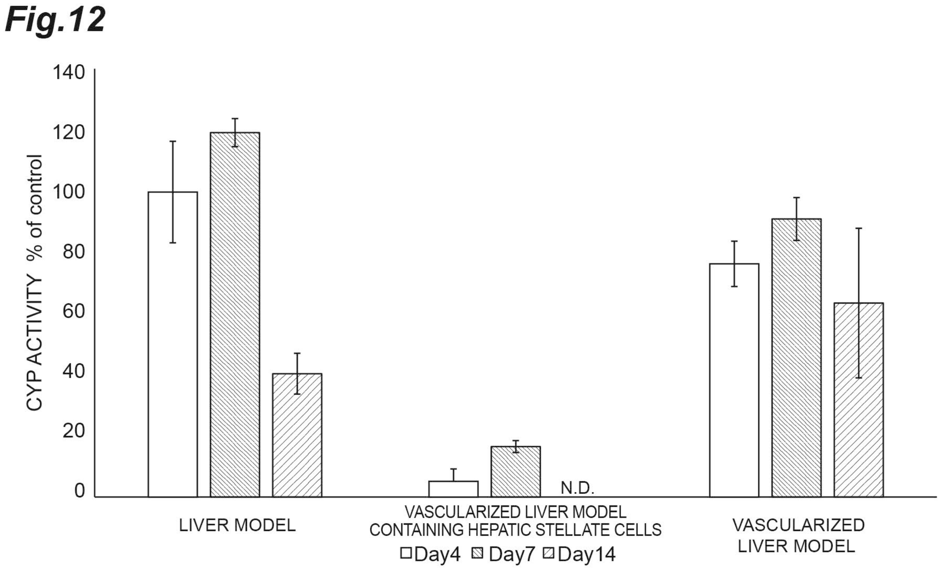

On Day 4, Day 7, and Day 14 of culturing of the cell structures, CYP3A4 enzyme activity in the cell structures was quantified using the P450-Glo (trademark) CYP3A4 Assay and Screening System. Seventy-two hours prior to measuring CYP3A4 activity (Day 1, Day 4, and Day 11), the medium was replaced with a medium including rifampicin, a compound that induces CYP3A4 expression, and the cell structures were cultured in this medium. Subsequently, CYP3A4 activity in each cell structure was measured using the aforementioned kit. This method involves the hydrolysis of luciferin substrate (a CYP3A4-specific substrate) taken up by the cells via CYP3A4, converting it into luciferin. By mixing with a luciferin detection reagent, luciferin is transformed into a luminescent substance, and the luminescence intensity is used to quantify the CYP3A4 activity of the cells. The results are illustrated in FIG. 12. In FIG. 12, samples for which activity could not be detected (luminescence intensity was below the detection limit) are indicated as "N.D."

As illustrated in FIG. 12, in the vascularized liver model and the vascular liver model containing hepatic stellate cells, the CYP3A4 activity was reduced on Day 14 of culturing. On the other hand, in the vascularized liver model, CYP3A4 activity was maintained even on Day 14 of culturing, indicating that the vascularized liver model maintains the expression of the CYP3A4 gene.

## Claims

1. A method for producing a cell structure, comprising:
a step of culturing a plurality of cells, comprising at least epithelial cells and vascular endothelial cells, in a medium comprising one or more growth factors involved in angiogenesis.

2. The method according to claim 1, wherein
the medium comprises two or more growth factors involved in angiogenesis, and
the two or more growth factors involved in angiogenesis comprise a vascular development factor and a vascular maintenance factor.

3. The method according to claim 1 or 2, wherein a content of the growth factor involved in angiogenesis in the medium is 10 ng/mL or more.

4. The method according to claim 1 or 2, wherein the step of culturing is performed under conditions that allow a medium component to be taken up from all directions of the cell structure.

5. The method according to claim 1 or 2, wherein the epithelial cells are hepatocytes and the cells do not comprise hepatic stellate cells.

6. The method according to claim 1 or 2, further comprising:
prior to the step of culturing, a step of obtaining a mixture comprising the plurality of cells and an extracellular matrix component.

7. The method according to claim 6, wherein the extracellular matrix components comprise collagen components.

8. The method according to claim 6, wherein the mixture further comprises a polymer electrolyte.

9. The method according to claim 8, wherein the polymer electrolyte comprises heparin.
